# EUROPÄISCHE PATENTSCHRIFT

(11) **EP 1 496 971 B1**
(45) Veröffentlichungstag und Bekanntmachung des Hinweises auf die Patenterteilung: **03.03.2010**
(21) Anmeldenummer: 03725068.5
(22) Anmeldetag: 22.04.2003
(51) Int. Cl.: A61M 25/00

(54) **BALLON-KATHETER**
BALLOON CATHETER
CATHETER A BALLONNET

(30) Priorität: 22.04.2002 DE 10217868
(43) Veröffentlichungstag der Anmeldung: 19.01.2005
(73) Patentinhaber: Abbott Laboratories Vascular Enterprises Limited, Dublin 2 (IE)
(72) Erfinder: JOERGENSEN, Ib, 72401 Haigerloch (DE); JEFFREY, Andrew, 72414 Rangendingen (DE); HA, Suk-Woo, CH-8246 Langwiesen (CH)
(74) Vertreter: Schmitz, Hans-Werner
(86) Internationale Anmeldenummer: PCT/EP2003/004149
(87) Internationale Veröffentlichungsnummer: WO 2003/089037

(56) Entgegenhaltungen:
- WO-A-92/03178
- US-A- 4 775 371
- US-A- 5 470 322
- US-B1- 6 217 566

## Beschreibung

Die Erfindung betrifft einen Ballon-Katheter gemäß dem Oberbegriff des Anspruches 1. Ein derartiger Katheter ist aus der US-A-5 470 322 bekannt.

Katheter, die als "over-the-wire-Katheter" bezeichnet werden, weisen einen Katheterschaft auf, der an seinem distalen Ende mit einem inflatierbaren Ballon versehen ist. Am gegenüberliegenden proximalen Ende ist üblicherweise ein Anschlussstück angeordnet. Ferner weist der bekannte Katheter einen Führungsdraht auf, der durch ein Führungsdrahtlumen des Katheterschaftes vom proximalen Ende bis zum distalen Ende und durch den Ballon hindurch verläuft. Schließlich ist im Katheterschaft ein Inflations- bzw. Deflationslumen vorgesehen, das vom proximalen Ende des Katheterschaftes bis zum Ballon verläuft und durch den Luft oder Flüssigkeit zum Ausdehnen des Ballons zugeführt und wieder abgeführt werden kann.

Soll ein derartiger Ballon-Katheter in eine Herzarterie eingeführt werden, wird zunächst ein äußerer Führungskatheter über die Aorta, üblicherweise vom Oberschenkel aus, mit ihrer gekrümmten Spitze bis zur zu behandelnden Herzarterie vorgeschoben, in der beispielsweise eine Stenose vorhanden ist, die mit dem Ballon-Katheter aufgeweitet und gegebenenfalls mit Hilfe eines gleichzeitig zu implantierenden Stents stabilisiert werden soll. Die Spitze des Führungskatheters wird hierbei temporär in dem Bereich der Abzweigung der zu behandelnden Herzarterie vom Aortabogen aus festgelegt. In den Führungskatheter wird anschließend der Führungsdraht eingeführt, bis dessen Spitze die im Herzkatheter befindliche Stenose passiert hat. Anschließend wird der Katheterschaft des Ballon-Katheters über den Führungsdraht in die Aorta und das zu behandelnde Gefäß eingeführt, bis der Ballon im Bereich der Stenose zu liegen kommt.

Bei dieser Art von Ballon-Katheter muss der Führungsdraht eine in etwa doppelte Länge des Katheters aufweisen, da der Katheter außerhalb des Körpers des Patienten zunächst auf den Führungsdraht aufgefädelt werden muss.

Bei einer weiteren, an sich bekannten Ballon-Katheter-Konstruktion ist ein Katheterschaft vorgesehen, der einen vom Anschlussstück ausgehenden, aus Metall bestehenden Schaftabschnitt aufweist, der bis auf das Inflationsvolumen ein Vollquerschnitt ist. An diesen (auch als Hypotube bezeichneten) Abschnitt schließt sich ein Kunststoffabschnitt bis zum Ballon an, in dem neben dem Inflationsvolumen ein Führungsdrahtvolumen vorgesehen ist, dass jedoch vor dem aus Stahl bestehenden Schaftabschnitt eine relativ kurz vor dem Ballon liegende Austrittsöffnung aufweist. Dies ermöglicht die Verwendung wesentlich kürzerer Führungsdrähte. Darüber hinaus ist diese Katheterart auf Grund des aus Stahl bestehenden, im proximalen Bereich liegenden Schaftabschnittes mit einer höheren Steifigkeit versehen, die die sogenannte "pushability" verbessert.

Es ist Aufgabe der vorliegenden Erfindung, ein Ballon-Katheter der im Oberbegriff des Anspruches 1 angegebenen Art zu schaffen, der es ermöglicht, einen over-the-wire Katheter mit einer erhöhten "pushability" zu schaffen.

Die Lösung dieser Aufgabe erfolgt durch die Merkmale des Anspruches 1.

Dadurch, dass der erfindungsgemäße Katheter einen in seiner Länge wählbaren und vom proximalen Ende aus verlaufenden Katheterschaftabschnitt aufweist, der mit einem Verstärkungsrohr versehen ist, ergibt sich zunächst eine deutliche verbesserte "pushability" und der Vorteil, dass die Reibung zwischen dem Führungsdraht und dem Katheterschaft deutlich verringert werden kann. Dies vereinfacht wesentlich das Handling des erfindungsgemäßen Katheters. Es ist vorzugsweise auch möglich, den Innenumfang eines Verstärkungsrohres aus Metall, vorzugsweise Stahl, mit einer die Reibung weiter vermindernden Beschichtung, beispielsweise aus PTFE zu versehen.

Die Unteransprüche haben vorteilhafte Weiterbildungen der Erfindung zum Inhalt.

Bei einer besonders bevorzugten Ausführungsform des Erfindungsgemäßen Katheters mit einem aus Metall bestehenden Verstärkungsrohr kann zur Verminderung der statischen und dynamischen Reibung für den Führungsdraht das Metallrohr innen mit einer Beschichtung ausgekleidet sein. Die Beschichtung kann aus Kunststoff, wie beispielsweise Polyethylen, PTFE oder Teflon bestehen.

Zur Beschichtung ist es möglich, einen Kunstoff-Schlauch, z. B. aus Polyethylen, mit einem etwas größeren Durchmesser als der Innenausnehmung des Verstärkungsrohres auf einem Stilett mit oder ohne Hitze-Einwirkung aufzuziehen, bis der Außendurchmesser des Schlauches geringer ist als der Innendurchmesser des Metall-Verstärkungsrohres. Der Schlauch wird mit dem Stilett ins Innere eingeführt und durch longitudiale Erwärmung durch die entsprechende Ausdehnung des Schlauches auf die Innenwand des Verstärkungsrohres aufgebracht. Die Enden des Schlauches werden nach Entfernung des Stiletts abgedreht oder abgeschnitten.

Bei einer weiteren besonders bevorzugten Ausführungsform kann der Kunststoff-Schlauch vor dem Ziehen durch Bestrahlung vernetzt (cross-linking) werden, um eine bessere Rückdehnung des Schlauches im Verstärkungsrohr zu erreichen.

Bei einer weiteren Ausführungsform wird die Oberfläche des Kunststoff-Schlauches vor der Einbringung in das Metall-Verstärkungsrohr zur Erhöhung der Haftfähigkeit an der Innenwand entsprechend modifiziert, z. B. durch PlasmaBehandlung oder Corona-Behandlung.

Weiterhin kann der Schlauch für die Kunststoffbeschichtung aus beispielsweise Polyethylen coextrudiert sein und mit einer äußeren Klebeschicht aus beispielsweise Ethylenvinylacetat (EVA) oder Nylon versehen werden.

Bei einer alternativen Ausführungsform wird der Kunststoff-schlauch durch erhitzte Pressluft an die Innenwand des Metall-Verstärkungsrohres angeschmolzen.

Zur besseren Verbindung des Verstärkungsrohres mit der Kunststoffbeschichtung kann zusätzlich Klebstoff zwischen die Innenwand des Verstärkungsrohres und dem Schlauch über die gesamte Länge des Verstärkungsrohres oder an den Enden desselben eingebracht werden.

Bei einer weiteren Ausführungsform ist es möglich, die Innenbeschichtung über den Bereich des Metall-Verstärkungsrohres auch in den angrenzenden Bereich des Kunststoffrohres übergehen zu lassen. Im extremen Fall ist es möglich, die gesamte Innenwand des Metall-Verstärkungsrohres und des anschließenden Kunststoffrohres mit der Beschichtung zu versehen.

Insgesamt dienen die Beschichtungen in allererster Linie dafür, die Reibung zwischen der Innenwand des jeweiligen Rohrabschnittes und dem Führungsdraht zu vermindern.

Als Knickschutz ist es möglich, ferner im Übergangsbereich zwischen dem metallischen Verstärkungsrohr und dem Kunststoffrohr einen Nylonschlauch-Überzug vorzusehen.

Weitere Einzelheiten, Merkmale und Vorteile der Erfindung ergeben sich aus nachfolgender Beschreibung der Zeichnung.

Es zeigt:
- Fig. 1: eine schematisch stark vereinfachte Prinzipdarstellung des erfindungsgemäßen Katheters,
- Fig. 2: eine Schnittdarstellung entlang der Linie A-A,
- Fig. 3: eine Schnittdarstellung entlang der Linie B-B, und
- Fig. 4: die Einzelheit X in vergrößerter Darstellung.

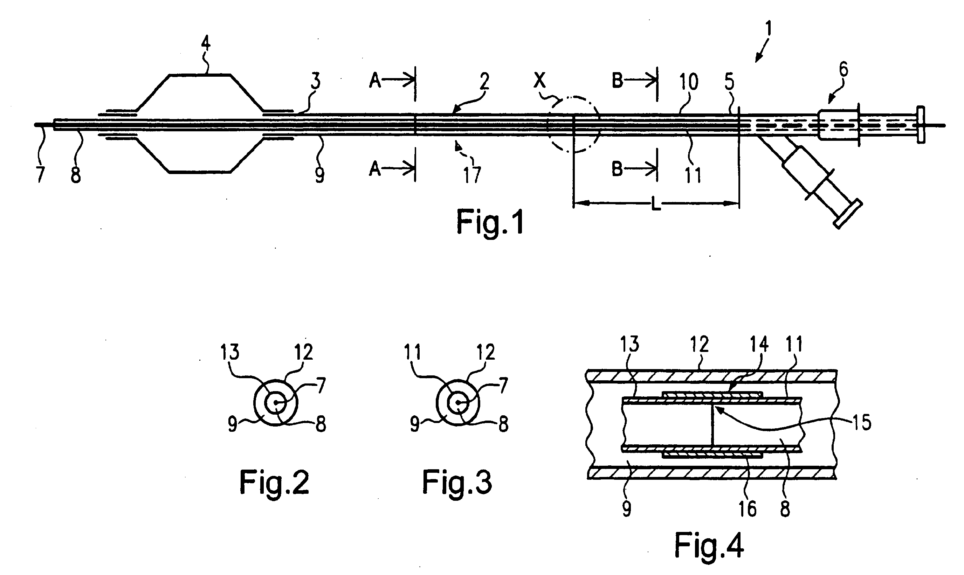

In Fig. 1 ist ein Ballon-Katheter 1 dargestellt. Der Katheter 1 weist einen Katheterschaft 2 auf. Am distalen Ende 3 des Katheterschaftes 2 ist ein inflatierbarer Ballon 4 vorgesehen. Am proximalen Ende 5 des Katheterschaftes 2 ist ein Anschlussstück 6 vorgesehen.

Ferner zeigt Fig. 1 einen Führungsdraht 7, der durch das Anschlussstück 6 hindurch in ein Führungsdrahtlumen 8 des Katheterschaftes 2 vom proximalen Ende 5 bishin zum distalen Ende 3 und durch den Ballon 4 hindurch verläuft.

Ferner ist ein Inflations- bzw. Deflationslumen 9 vorgesehen, dass vom Anschlussstück 6 durch den Katheterschaft 2 hindurch bis zum Ballon 4 verläuft.

Wie in Fig. 1 dargestellt, weist der Katheterschaft 2 ein vom proximalen Ende 5 aus verlaufenden Abschnitt 10 auf, der mit einem Verstärkungsrohr 11 aus Metall oder Kunststoff versehen ist. Ferner ist es möglich, dass das Verstärkungsrohr aus Metall besteht und mit einer Innenbeschichtung aus Kunststoff, vorzugsweise zur Reibungsverminderung, versehen ist.

Die Fig. 2 zeigt eine Querschnittsdarstellung, die die konzentrische Anordnung der zuvor beschriebenen Lumen 8 und 9 bzw. der diese begrenzenden Rohre 12 und 13 verdeutlicht. In diesem Abschnitt des Katheterschaftes 2 sind die Rohre 12 und 13 als Kunststoffrohre ausgebildet, wobei das Lumen 9 zwischen dem Rohr 12 und 13 und das Lumen 8 im Rohr 13 liegt, durch das der Führungsdraht 7 verläuft.

Fig. 3 verdeutlicht die Schnittdarstellung gemäß B-B in Fig. 1. In diesem Bereich ist das aus Metall, vorzugsweise Stahl, wie Edelstahl, bestehende Verstärkungsrohr 11 zu sehen, das vom Kunststoffrohr 12 zur Begrenzung des Lumens 9 in diesem Abschnitt umgeben ist.

Fig. 4 verdeutlicht die Einzelheit X in Fig. 1.

Das äußere Rohr 12 umgibt einen Übergangsabschitt 15 zwischen dem Verstärkungsrohr 11 und dem sich daran anschließenden inneren Kunststoffrohr 13. Dieser Übergangsabschnitt 15 ist mit einem Knickschutz 14 versehen, der im Beispielsfalle als Hülse 16 ausgebildet ist.

## Patentansprüche

1. Ballon-Katheter (1)
- mit einem Katheterschaft (2), an dessen distalen Ende (3) ein inflatierbarer Ballon (4) angeordnet ist und an dessen proximalen Ende (5) ein Anschlussstück (6) angeordnet ist;
- mit einem Führungsdrahtlumen (8), durch das ein Führungsdraht (7) hindurchführbar ist und das vom proximalen Ende (5) bis zum distalen Ende (3) des Katheterschaftes (2) und den Ballon (4) verläuft; und
- mit einem Inflations- bzw. Deflationslumen (9), das vom proximalen Ende (5) des Katheterschaftes (2) bis zum Ballon (4) verläuft,
- wobei ein in seiner Länge (L) wählbarer, vom proximalen Ende (5) aus verlaufender Abschnitt (10) des Katheterschafts (2) mit zumindest einem Verstärkungsrohr (11) versehen ist,
**dadurch gekennzeichnet,**
- **dass** das Führungsdrahtlumen (8) von einem Rohr (11, 13) umgeben ist, das im in seiner Länge (L) wählbaren Abschnitt (10) vom Verstärkungsrohr (11) und im sich auf dessen distaler Seite anschliessender Abschnitt von einem Rohr (13) aus Kunststoff gebildet ist, und
- **dass** das Inflations- bzw. Deflationslumen (9) zwischen dem das Führungsdrahtlumen (8) umgebenden Rohr (11, 13) und einem äußeren Rohr (12) angeordnet ist.

2. Katheter nach Anspruch 1, **dadurch gekennzeichnet, dass** das Verstärkungsrohr (11) aus Metall, insbesondere aus nicht rostendem Stahl besteht.

3. Katheter nach Anspruch 1 oder 2, **dadurch gekennzeichnet, dass** ein Übergangsbereich (15) zwischen dem Verstärkungsrohr (11) und einem zweiten Katheterschaftabschnitt (13) mit einem Knickschutz (14) versehen ist.

4. Katheter nach Anspruch 3, **dadurch gekennzeichnet, dass** der Knickschutz (14) als Hülse (14) ausgebildet ist, die über den Übergangsbereich hinweg auf dem Katheterschaft (2) angeordnet ist.

5. Katheter nach Anspruch 3, **dadurch gekennzeichnet, dass** der Knickschutz (14) als Metallfeder ausgebildet ist, die in der Wand des Katheterschaftes (2) den Übergangsbereich überbrückt.

6. Katheter nach Anspruch 3, **dadurch gekennzeichnet, dass** der Knickschutz (14) als Metallfeder ausgebildet ist, die im Inflations- bzw. Deflationslumen (9) angeordnet ist.

7. Katheter nach einem der Ansprüche 1 bis 6, **dadurch gekennzeichnet, dass** auf dem Ballon (4) ein Stent platzierbar ist.

8. Katheter nach Anspruch 1, **dadurch gekennzeichnet, dass** die Rohre (11, 13 bzw. 12) des Katheterschafts (2) konzentrisch angeordnet sind und zur Erhöhung der "pushability" miteinander verbunden sind.

9. Katheter nach Anspruch 3, **dadurch gekennzeichnet, dass** der Knickschutz (14) als Nylonschlauch-Überzug ausgebildet ist.

10. Katheter nach einem der Ansprüche 1 oder 3 bis 8, **dadurch gekennzeichnet, dass** das Verstärkungsrohr (11) aus Kunststoff besteht.

11. Katheter nach Anspruch 2, **dadurch gekennzeichnet, dass** das Verstärkungsrohr (11) aus Metall mit einer Innenbeschichtung aus Kunststoff versehen ist.

## Claims

1. A balloon catheter (1), comprising
- a catheter shaft (2), at the distal end (3) of which an inflatable balloon (4) is arranged and at the proximal end (5) of which a connector (6) is arranged;
- a guide wire lumen (8) through which a guide wire (7) can be passed and which extends from the proximal end (5) to the distal end (3) of the catheter shaft (2) and the balloon (4); and
- an inflation or deflation lumen (9) extending from the proximal end (5) of the catheter shaft (2) to the balloon (4),
- wherein a section (10) of the catheter shaft (2) is provided with at least one reinforcing pipe (11), said section having a selectable length (L) and extending from the proximal end (5),
**characterized in**
**that** the guide wire lumen (8) is surrounded by a pipe (11, 13) which is formed by the reinforcing pipe (11) in its section (10) having a selectable length (L) and by a pipe (13) made of plastics in the section adjacent at the distal side thereof, and
**that** the inflation or deflation lumen (9) is arranged between the pipe (11, 31) surrounding the guide wire lumen (8) and an external pipe (12).

2. The catheter of claim 1, **characterized in that** the reinforcing pipe (11) is made of metal, in particular of stainless steel.

3. The catheter of claim 1 or 2, **characterized in that** a transition area (15) between the reinforcing pipe (11) and a second catheter shaft section (13) is provided with an anti-flex protection (14).

4. The catheter of claim 3, **characterized in that** the anti-flex protection (14) is formed as a sleeve (14) which is arranged on the catheter shaft (2) beyond the transition area.

5. The catheter of claim 3, **characterized in that** the anti-flex protection (14) is formed as a metal spring which bridges the transition area in the wall of the catheter shaft (2).

6. The catheter of claim 3, **characterized in that** the anti-flex protection (14) is formed as a metal spring which is arranged in the inflation or deflation lumen (9).

7. The catheter of one of claims 1 to 6, **characterized in that** a stent can be disposed on the balloon (4).

8. The catheter of claim 1, **characterized in that** the pipes (11, 13 or 12) of the catheter shaft (2) are arranged concentrically and are connected to each other in order to enhance the pushability.

9. The catheter of claim 3, **characterized in that** the anti-flex protection (14) is formed as a nylon tube coating.

10. The catheter of one of claims 1 or 3 to 8, **characterized in that** the reinforcing pipe (11) is made of plastics.

11. The catheter of claim 2, **characterized in that** the reinforcing pipe (11) made of metal is provided with an internal lining made of plastics.

## Revendications

1. Cathéter à ballonnet (1) comportant
- un corps de cathéter (2) à l'extrémité distale (3) duquel est aménagé un ballonnet gonflable (4) et à l'extrémité proximale (5) duquel est aménagé un élément de raccordement (6),
- un lumen de fil de guidage (8) par lequel un fil de guidage (7) peut être conduit et qui s'étend de l'extrémité proximale (5) à l'extrémité distale (3) du corps de cathéter (2) et le ballonnet (4) ; et
- un lumen de gonflage ou de dégonflage (9) qui s'étend de l'extrémité distale (5) du corps de cathéter (2) jusqu'au ballonnet (4),
- un segment (10) du corps de cathéter (2) pouvant évoluer sur sa longueur (L) de façon sélectionnable à partir de l'extrémité proximale (5) étant doté au moins d'un tube de renforcement (11),
**caractérisé en ce que**
- le lumen du fil de guidage (8) est entouré d'un tube (11, 13) qui est en matière plastique dans le segment pouvant évoluer sur sa longueur (L) de façon sélectionnable du tube de renforcement (11) et dans le segment se raccordant à son côté distal, d'un tube (13), et
- le lumen de gonflage ou de dégonflage (9) est disposé entre le tube (11, 13) entourant le lumen du fil de guidage (8) et un tube externe (12).

2. Cathéter selon la revendication 1, **caractérisé en ce que** le tube de renforcement (11) est constitué de métal, en particulier de métal antirouille.

3. Cathéter selon la revendication 1 ou 2, **caractérisé en ce qu'**une zone de transition (15) entre le tube de renforcement (11) et un deuxième segment de cathéter (13) est dotée d'une protection contre le pliage (14).

4. Cathéter selon la revendication 3, **caractérisé en ce que** la protection contre le pliage (14) est configurée sous la forme d'une gaine (14) qui est disposée sur la zone de transition s'étendant sur le corps du cathéter (2).

5. Cathéter selon la revendication 3, **caractérisé en ce que** la protection contre le pliage (14) est configurée sous la forme d'un ressort métallique qui couvre la zone de transition dans la paroi du corps de cathéter (2).

6. Cathéter selon la revendication 3, **caractérisé en ce que** la protection contre le pliage (14) est configurée sous la forme d'un ressort métallique qui est disposé dans le lumen de gonflage ou dégonflage.

7. Cathéter selon l'une des revendications 1 à 6, **caractérisé en ce qu'**un stent peut être placé sur le ballonnet (4).

8. Cathéter selon la revendication 1, **caractérisé en ce que** les tubes (11, 13 ou 12) du corps de cathéter (2) sont disposés de façon concentrique et sont reliés pour augmenter la capacité de poussage les uns avec les autres.

9. Cathéter selon la revendication 3, **caractérisé en ce que** la protection contre le pliage (14) est configurée sous la forme d'un revêtement de nylon.

10. Cathéter selon l'une des revendications 1 ou 3 à 8, **caractérisé en ce que** le tube de renforcement (11) est en matière plastique.

11. Cathéter selon la revendication 2, **caractérisé en ce que** le tube de renforcement (11) est en métal et comporte un revêtement interne en matière plastique.
